# EUROPEAN PATENT APPLICATION

(11) **EP 3 943 601 A1**
(43) Date of publication of application: **26.01.2022**
(21) Application number: 20773279.3
(22) Date of filing: 27.02.2020
(51) Int. Cl.: C12N 15/11

(54) **PROTEIN TRANSLATION USING CIRCULAR RNA AND APPLICATION THEREOF**

(30) Priority: 20.03.2019 CN 201910214234
(71) Applicant: Shanghai Institute of Nutrition and Health, Chinese Academy of Sciences, Shanghai 200031 (CN)
(72) Inventor: WANG, Zefeng, Shanghai 200031 (CN); YANG, Yun, Shanghai 200031 (CN); FAN, Xiaojuan, Shanghai 200031 (CN)
(74) Representative: Rondano, Davide
(86) International application number: PCT/CN2020/077026
(87) International publication number: WO 2020/186991

(57) **Abstract**

Provided are a circular RNA construct and application thereof. The circular RNA construct has a structure represented by formula I in the 5'-3' direction: TI-Z1-Z2 (I), wherein TI is a translation initiation element, Z1 is an expression cassette for exogenous protein expression, and Z2 is nothing or another element.

## Description

### Technical field

The present invention relates to the field of biotechnology, in particular to the use of circular RNA for protein translation and its application.

### Background

Protein is the most important biological macromolecule in the organism, and its mutation or abnormal expression can cause disease. Therefore, the corresponding diseases can be treated by protein substitution or expression. Common protein substitution or expression therapies include ribonucleic acid (DNA) vector-based delivery systems, deoxyribonucleic acid (RNA) vector-based delivery systems, and protein delivery systems. These methods all need to produce protein through messenger RNA translation. The common translation initiation method in eukaryotes is cap-dependent translation, which mainly uses the translation initiation factor to recognize the special cap structure at the 5' end of the messenger RNA to initiate translation. This type of translation method only exists in linear messenger RNA. In addition, there is a type of cap-independent translation initiation, which mainly initiates translation through the interaction between specific protein factors and RNA elements. This type of translation can be initiated in linear or circular RNA. Common cap-independent translation initiation elements are some elements with specific secondary structure in viral RNA, and they can use the host cell's translation system to express the proteins they need. For example, the internal ribosome entry site (IRES) element contained in RNA such as encephalomyocarditis virus or hepatitis C virus.

Circular RNA is a type of single-stranded closed-loop RNA that is different from linear RNA. Because of its structural specificity, it is not easily degraded by exonuclease, and it is more stable than linear RNA. Therefore, the expression of protein through circular RNA translation has the characteristics of more continuous and long-term effect, and is an important means to replace linear RNA translation. However, because the translation of circular RNA can only use the trans of cap-independent translation, how to design and select a suitable cap-independent translation initiation element is a key technology for this application. A common method is to use viral IRES to initiate the translation of circular RNA. However, there may be immune rejection in the host due to the pathogenic virus RNA, the RNA elements derived from viruses basically contain complex RNA secondary structures and moreover, the longer sequence limits the construction of viral IRES-based expression systems and later gene therapy applications.

Therefore, there is an urgent need in the art to develop a cap-independent translation initiation element of non-viral origin.

### Summary of the invention

The purpose of the present invention is to provide a cap-independent translation initiation element of non-viral origin.

In a first aspect of the present invention, it provides a circular RNA construct which has a structure as shown in Formula I from the 5'-3' direction:

TI-Z1-Z2 (I)

wherein
TI is a translation initiation element;
Z1 is an expression cassette for expressing a foreign protein;
Z2 is none or an other component;
and, each "-" is a bond or a nucleotide connection sequence;
wherein, the length of the TI element is 6-30 nt, preferably, 8-24 nt, more preferably, 10-20 nt;
in the TI element, the content of A is ≥35%, preferably, ≥45%, more preferably, ≥60%;
in the TI element, the content of T is ≥20%, preferably, ≥30%, more preferably, ≥50%;
in the TI element, the content of A+T is ≥65%, preferably, ≥80%, more preferably, ≥90%;
in the TI element, the content of G is ≤35%, preferably, ≤25%, and more preferably, ≤10%.

In another preferred embodiment, the circular RNA construct is a circular messenger RNA construct.

In another preferred embodiment, the content of A in the TI element is 35-100%, preferably, 45-100%, more preferably, 60-100%.

In another preferred embodiment, the content of T in the TI element is 20-100%, preferably, 30-100%, more preferably, 50-100%.

In another preferred embodiment, the content of A+T in the TI element is 65-100%, preferably, 80-100%, more preferably, 90-100%.

In another preferred embodiment, the content of G in the TI element is 0-35%, preferably, 0-25%, more preferably, 0-10%.

In another preferred embodiment, the TI element contains one or more nucleotide sequences selected from the group consisting of as shown in Table 1:

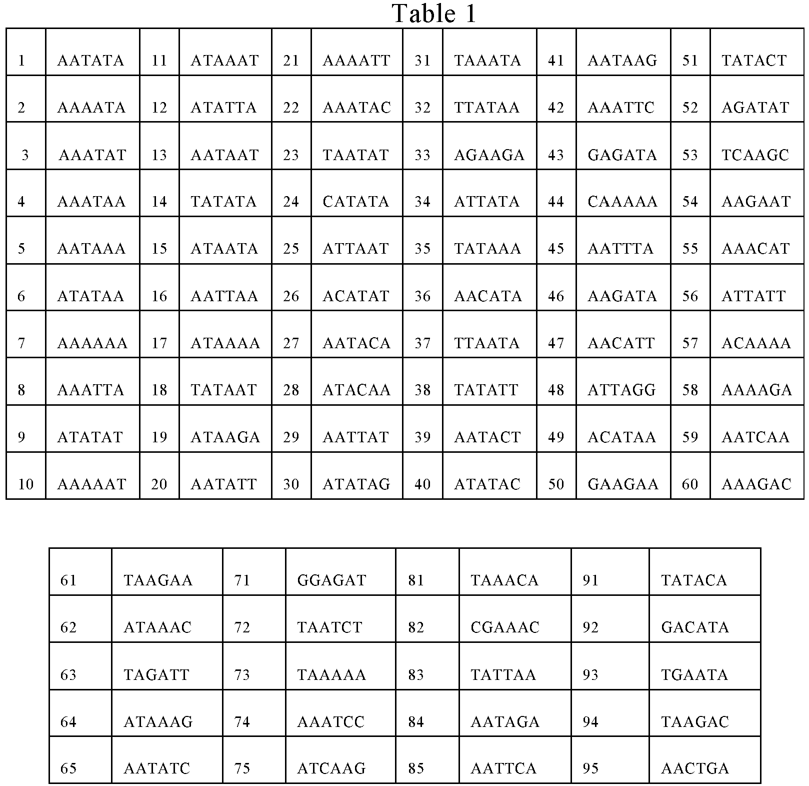

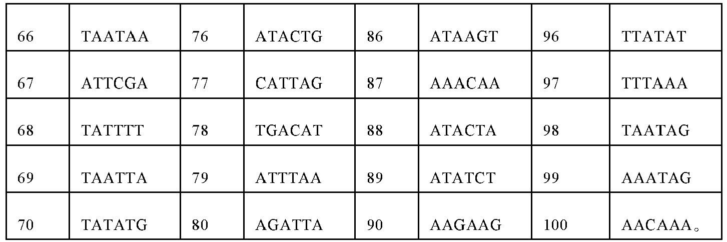

In another preferred example, the TI element has 1-24 (preferably 1-15, more preferably 1-10, more preferably, 1-6) nucleotides added to the 5' end and/or 3' end of the nucleotide sequence as shown in Table 1, and has the function of a TI element.

In another preferred embodiment, the coding sequence of the TI element is selected from the group consisting of;
(a) a polynucleotide whose sequence is shown in SEQ ID NO.: 1-40;
(b) a polynucleotide whose nucleotide sequence is more than 75% (preferably ≥85%, more preferably ≥90% or ≥95% or ≥98% or ≥99%) homologous to the sequence as shown in SEQ ID NO.: 1-40;
(c) a polynucleotide has 1-18 (preferably 1-10, more preferably 1-6) nucleotides truncated or added at the 5' end and/or 3' end of the polynucleotide as shown in SEQ ID NO.: 1-40;
(d) a polynucleotide complementary to any of the polynucleotides as described in (a) to (c).

In another preferred embodiment, the TI element has a sequence as shown in SEQ ID NO.: 1-40.

In another preferred embodiment, the coding sequence of the TI element is shown in SEQ ID NO.: 1-40.

In another preferred embodiment, the Z1 element contains a stop codon.

In another preferred embodiment, the Z1 element does not contain a stop codon.

In another preferred embodiment, the coding sequence of the foreign protein is derived from a prokaryotic organism or a eukaryotic organism.

In another preferred embodiment, the coding sequence of the foreign protein is derived from animals, plants, and pathogens.

In another preferred embodiment, the coding sequence of the foreign protein is derived from mammals, preferably primates, rodents, including humans, mice, and rats.

In another preferred embodiment, the encoding sequence of the foreign protein is selected from the group consisting of an exogenous DNA encoding luciferin protein or luciferase (such as firefly luciferase), green fluorescent protein, yellow fluorescent protein, aminoacyl tRNA synthetase, glyceraldehyde-3-phosphate dehydrogenase, catalase, actin, variable regions of antibodies, DNA of luciferase mutants, and a combination thereof.

In another preferred embodiment, the foreign protein is selected from the group consisting of: luciferin, or luciferase (such as firefly luciferase), green fluorescent protein, yellow fluorescent protein, aminoacyl tRNA synthetase, glyceraldehyde- 3-phosphate dehydrogenase, catalase, actin, variable regions of antibodies, luciferase mutations, α-amylase, enterocin A, hepatitis C virus E2 glycoprotein, insulin precursor, interferon alpha A, interleukin-1 beta, lysozyme, serum albumin, single-chain antibody fragment (scFV), transthyretin, tyrosinase, xylanase, and a combination thereof.

In another preferred embodiment, the Z2 element is selected from the group consisting of PolyA, multiple cloning site, aptamer, miRNA binding site, translation enhancement element, and a combination thereof.

In another preferred embodiment, one or more adenines (A) of the TI element are methylated.

In another preferred embodiment, the sequence of the circular RNA construct is as shown in SEQ ID NO.: 61.

In a second aspect of the present invention, it provides a vector containing an expression cassette of the construct according to the first aspect of the present invention.

In another preferred embodiment, the expression cassette contains a first intron and a second intron.

In another preferred embodiment, the first intron and the second intron are completely complementary or not completely complementary.

In another preferred embodiment, the vector has a sequence as shown in SEQ ID NO.:62.

In another preferred embodiment, the sequence of the first intron is shown in SEQ ID NO.: 63.

In another preferred embodiment, the sequence of the second intron is shown in SEQ ID NO.: 64.

In a third aspect of the present invention, it provides a genetically engineered cell in which the nucleic acid construct of the first aspect of the present invention is integrated at one or more sites of the genome of the genetically engineered cell, or the genetically engineered cell contains the vector according to the second aspect of the present invention.

In another preferred embodiment, the genetically engineered cells include prokaryotic cells and eukaryotic cells.

In another preferred embodiment, the eukaryotic cells include higher eukaryotic cells.

In another preferred embodiment, the genetically engineered cells are selected from the group consisting of human-derived cells (such as HeLa cells), Chinese hamster ovary cells, insect cells, wheat germ cells, rabbit reticulocytes, yeast cells, and combinations thereof.

In another preferred embodiment, the genetically engineered cell is a yeast cell.

In another preferred embodiment, the yeast cell is selected from the group consisting of Saccharomyces cerevisiae, the yeast of Kluyveromyces sp., and a combination thereof.

In another preferred embodiment, the yeast of the Kluyveromyces sp. is selected from the group consisting of Kluyveromyces lactis, Kluyveromyces marxianus, Kluyveromyces dobriella, and a combination thereof.

In a fourth aspect of the present invention, it provides a reaction system, comprising:
(a) a construct according to the first aspect of the present invention;
(b) other components required for the reaction, and the other components are selected from the group consisting of splicesomes, ribosomes, translation initiation factor EIF4G2, translation initiation factor EIF4A, translation initiation factor EIF4B, and a combination thereof.

In another preferred embodiment, the reaction system further includes YTHDF3, PABPC1, and/or hnRNPA1 protein.

In another preferred embodiment, the reaction system is an in vitro reaction system.

In a fifth aspect of the present invention, it provides a method for synthesizing protein in vitro, comprising the steps:
(i) providing the reaction system according to the fourth aspect of the present invention;
(ii) under a suitable condition, incubating the synthesis system of step (i) for a period of time T1, thereby synthesizing the protein.

In another preferred embodiment, the method further includes: (iii) optionally separating or detecting the protein from the in vitro reaction system.

In another preferred embodiment, in the step (ii), the reaction temperature is 25-42°C, preferably, 30-40°C, more preferably, 35-37°C.

In another preferred embodiment, in the step (ii), the reaction time T1 is 1 hour to 20 hours, preferably, 2 hours to 12 hours, more preferably, 3 hours to 6 hours.

In a sixth aspect of the present invention, it provides a kit for in vitro protein synthesis, comprising:
(k1) a first container, and the structure according to the first aspect of the present invention located in the first container;
(k2) a second container, and other components required for the reaction located in the second container, the other components are selected from the group consisting of spliceosome, ribosome, translation initiation factor EIF4G2, translation initiation factor EIF4A, the translation initiation factor EIF4B, and a combination thereof; and
(kt) label or instructions.

In another preferred embodiment, the first container and the second container are the same container or different containers.

In another preferred embodiment, the kit further includes optionally one or more containers selected from the group consisting of:
(k3) a third container, and the YTHDF3, PABPC1, and/or hnRNPA1 protein located in the third container.

In a seventh aspect of the present invention, it provides a use of a construct according to the first aspect of the present invention, a vector according to the second aspect of the present invention, a genetically engineered cell according to the third aspect of the present invention, and a reaction system according to the fourth aspect of the present invention or the kit according to the sixth aspect of the present invention for high-throughput in vitro protein synthesis.

It should be understood that, within the scope of the present invention, each technical feature of the present invention described above and in the following (as examples) may be combined with each other to form a new or preferred technical solution, which is not listed here due to space limitations.

### Description of Figure

Figure 1 shows the different cell populations screened by flow cytometry.
Figure 2 shows the western blot to detect the activity of the characteristic sequence of the translation initiation element.
Figure 3 shows the western blot to detect the activity of translation initiation elements produced by anti-learning.

### Detailed Description

After extensive and in-depth research, through a large number of screenings and explorations, a specific translation initiation element is unexpectedly screened for the first time. The translation initiation element has high translation activity. Inserting the translational initiation element of the present invention into a circular RNA expression vector can significantly enhance translation efficiency both in vivo and in vitro. On this basis, the present inventor has completed the present invention.

### First intron

In the present invention, the 3' end of the first intron contains a acceptor splicing site, which contains a cis element (50bp-300bp in length) that is paired with the second intron.

The sequence is shown as follows:

GACTGAACATGGAGGAATTGAGGTTGGGTATTTCCCCTGAGGTAGGAAA
AAGGCTGGGTCAGTTTCCCGTTAGCCGTCAAGTCCTCATCACATCTTTAA
GCCTTCCATGCAGGATAAAGGGCTGCAGAGCTATTTTCAAATTGACATCA
AACTGGATTTCTGTTGACTTCGTCTTCCCTTTTTAAGGTCCACAGAAGAAG
ATGGGAAGGAAAGAAGTCTGAGGGCATCTTATTTGCACTCCGCTGTCATT
TCTAAGGAAGGCCTTTAATGCCAAATTCTCATCTTTTATGTCCCCACTAAA
TCCTAAGGTTCTTGAACTTCTGATCAGACAGCCAAAAAATGAACCATCAA
CTAGCTTAACCTAACATATGTGAGGATAGAGGACTGGGACAGCTCTCTGG
GCCACTGGAGAGTCAGACAGGCCTGCCCTCTGTGTGACTTGACCGCGGTC
TCTTTCTTCCAG （SEQ ID NO.:63）

### Second intron

In the present invention, the 5' end of the second intron contains a donor splicing site, which contains a cis element (50bp-300bp in length) that is paired with the first intron.

The sequence is shown as follows:

GTAAGTCTCGACGGATCCCAAATAAGATGCCCTCAGACTTCTTTCCTTCCC
ATCTTCTTCTGTGGACCTTAAAAAGGAAGACGAAGTCAACAGAAATCCA
GTTTGATGTCAATTTGAAAATAGCTCTGCAGCCCTTTATCCTGCATGGAA
GGCTTAAAGATGTGATGAGGACTTGACGGCTAACGGAAACTGACCCAG
CCTTTTTCCTACCTCAGGGGAAATACCCAACCTCAATTCCTCCATGTTCAG
（SEQ ID NO.:64）

### Circular RNA construct

The first aspect of the present invention provides a circular RNA construct which has a structure as shown in Formula I from the 5'-3' direction:

TI-Z1-Z2 (I)

wherein
TI is a translation initiation element;
Z1 is an expression cassette for expressing a foreign protein;
Z2 is none or an other component;
and, each "-" is a bond or a nucleotide connection sequence;
wherein, the length of the TI element is 6-30 nt, preferably, 8-24 nt, more preferably, 10-20 nt;

In the TI element, the content of A is ≥35%, preferably, ≥45%, more preferably, ≥60%;

In the TI element, the content of T is ≥20%, preferably, ≥30%, more preferably, ≥50%;

In the TI element, the content of A+T is ≥65%, preferably, ≥80%, more preferably, ≥90%;

In the TI element, the content of G is ≤35%, preferably, ≤25%, and more preferably, ≤10%.

In a preferred embodiment, the content of A in the TI element is 35-100%, preferably, 45-100%, more preferably, 60-100%.

In a preferred embodiment, the content of T in the TI element is 20-100%, preferably, 30-100%, more preferably, 50-100%.

In a preferred embodiment, the content of A+T in the TI element is 65-100%, preferably 80-100%, more preferably 90-100%.

In a preferred embodiment, the content of G in the TI element is 0-35%, preferably, 0-25%, more preferably, 0-10%.

In the present invention, the selection of the coding sequence of the foreign protein is not particularly limited. Generally, the coding sequence of the foreign protein is selected from the group consisting of: an exogenous DNA encoding luciferin protein or luciferase (such as firefly luciferase), green fluorescent protein, yellow fluorescent protein, aminoacyl tRNA synthetase, glyceraldehyde-3-phosphate dehydrogenase, catalase, actin, variable regions of antibodies, DNA of luciferase mutants, and a combination thereof.

The coding sequence of the foreign protein can also encode a protein selected from the group consisting of α-amylase, enterocin A, hepatitis C virus E2 glycoprotein, insulin precursor, interferon alpha A, interleukin-1 beta, lysozyme, serum albumin, single-chain antibody fragment (scFV), transthyretin, tyrosinase, xylanase, and a combination thereof.

In addition, the nucleic acid construct of the present invention is circular. The nucleic acid construct of the present invention is single-stranded. The nucleic acid construct of the present invention is RNA.

In a preferred embodiment, the sequence of the circular RNA construct of the present invention is shown in SEQ ID NO.: 61.

Circular RNA sequence using GFP as an example:

5'-AAAAAAAAAATCATGGTGAGCAAGGGCGAGGAGCTGTTCACCGGGGTG
GTGCCCATCCTGGTCGAGCTGGACGGCGACGTAAACGGCCACAAGTTCAGC
GTGTCCGGCGAGGGCGAGGGCGATGCCACCTACGGCAAGCTGACCCTGAAG
TTCATCTGCACCACCGGCAAGCTGCCCGTGCCCTGGCCCACCCTCGTGACCA
CCCTGACCTACGGCGTGCAGTGCTTCAGCCGCTACCCCGACCACATGAAGCA
GCACGACTTCTTCAAGTCCGCCATGCCCGAAGGCTACGTCCAGGAGCGCAC
CATCTTCTTCAAGGACGACGGCAACTACAAGACCCGCGCCGAGGTGAAGTT
CGAGGGCGACACCCTGGTGAACCGCATCGAGCTGAAGGGCATCGACTTCAA
GGAGGACGGCAACATCCTGGGGCACAAGCTGGAGTACAACTACAACAGCCA
CAACGTCTATATCATGGCCGACAAGCAGAAGAACGGCATCAAGGTGAACTT
CAAGATCCGCCACAACATCGAGGACGGCAGCGTGCAGCTCGCCGACCACTA
CCAGCAGAACACCCCCATCGGCGACGGCCCCGTGCTGCTGCCCGACAACCA
CTACCTGAGCACCCAGTCCGCCCTGAGCAAAGACCCCAACGAGAAGCGCGA
TCACATGGTCCTGCTGGAGTTCGTGACCGCCGCCGGGATCACTCTCGGCATG
GACGAGCTGTACAAGTAA-3' （SEQ ID NO.:61）

In a preferred embodiment, the sequence of the circular RNA precursor (containing the first intron and the second intron) taking GFP as an example is as follows:

5'-GACTGAACATGGAGGAATTGAGGTTGGGTATTTCCCCTGAGGTAGGAAAA
AGGCTGGGTCAGTTTCCCGTTAGCCGTCAAGTCCTCATCACATCTTTAAGCC
TTCCATGCAGGATAAAGGGCTGCAGAGCTATTTCAAATTGACATCAAACTG
GATTTCTGTTGACTTCGTCTTCCCTTTTTAAGGTCCACAGAAGAAGATGGGA
AGGAAAGAAGTCTGAGGGCATCTTATTTGCACTCCGCTGTCATTTCTAAGGA
AGGCCTTTAATGCCAAATTCTCATCTTTTATGTCCCCACTAAATCCTAAGGTT
CTTGAACTTCTGATCAGACAGCCAAAAAATGAACCATCAACTAGCTTAACCT
AACATATGTGAGGATAGAGGACTGGACAGCTCTCTGGCCACTGGAGAGT
CAGACAGGCCTGCCCTCTGTGTGACTTGACCGCGGTCTCTTTCTTCCAGGAG
CGCACCATCTTCTTCAAGGACGACGGCAACTACAAGACCCGCGCCGAGGTG
AAGTTCGAGGGCGACACCCTGGTGAACCGCATCGAGCTGAAGGGCATCGAC
TTCAAGGAGGACGGCAACATCCTGGGGCACAAGCTGGAGTACAACTACAAC
AGCCACAACGTCTATATCATGGCCGACAAGCAGAAGAACGGCATCAAGGTG
AACTTCAAGATCCGCCACAACATCGAGGACGGCAGCGTGCAGCTCGCCGAC
CACTACCAGCAGAACACCCCCATCGGCGACGGCCCCGTGCTGCTGCCCGAC
AACCACTACCTGAGCACCCAGTCCGCCCTGAGCAAAGACCCCAACGAGAAG
CGCGATCACATGGTCCTGCTGGAGTTCGTGACCGCCGCCGGGATCACTCTCG
GCATGGACGAGCTGTACAAGTAAAAAAAAAAAAATCATGGTGAGCAAGGG
CGAGGAGCTGTTCACCGGGGTGGTGCCCATCCTGGTCGAGCTGGACGGCGA
CGTAAACGGCCACAAGTTCAGCGTGTCCGGCGAGGGCGAGGGCGATGCCAC
CTACGGCAAGCTGACCCTGAAGTTCATCTGCACCACCGGCAAGCTGCCCGTG
CCCTGGCCCACCCTCGTGACCACCCTGACCTACGGCGTGCAGTGCTTCAGCC
GCTACCCCGACCACATGAAGCAGCACGACTTCTTCAAGTCCGCCATGCCCGA
AGGCTACGTCCAGGTAAGTCTCGACGGATCCCAAATAAGATGCCCTCAGAC
TTCTTTCCTTCCCATCTTCTTCTGTGGACCTTAAAAAGGGAAGACGAAGTCA
ACAGAAATCCAGTTTGATGTCAATTTGAAAATAGCTCTGCAGCCCTTTATCC
TGCATGGAAGGCTTAAAGATGTGATGAGGACTTGACGGCTAACGGGAAACT
GACCCAGCCTTTTTCCTACCTCAGGGGAAATACCCAACCTCAATTCCTCCAT
GTTCAG-3'　（SEQ ID NO.:62）

In a preferred embodiment, the TI element of the present invention contains the nucleotide sequence selected from the group consisting of as shown in Table 1:

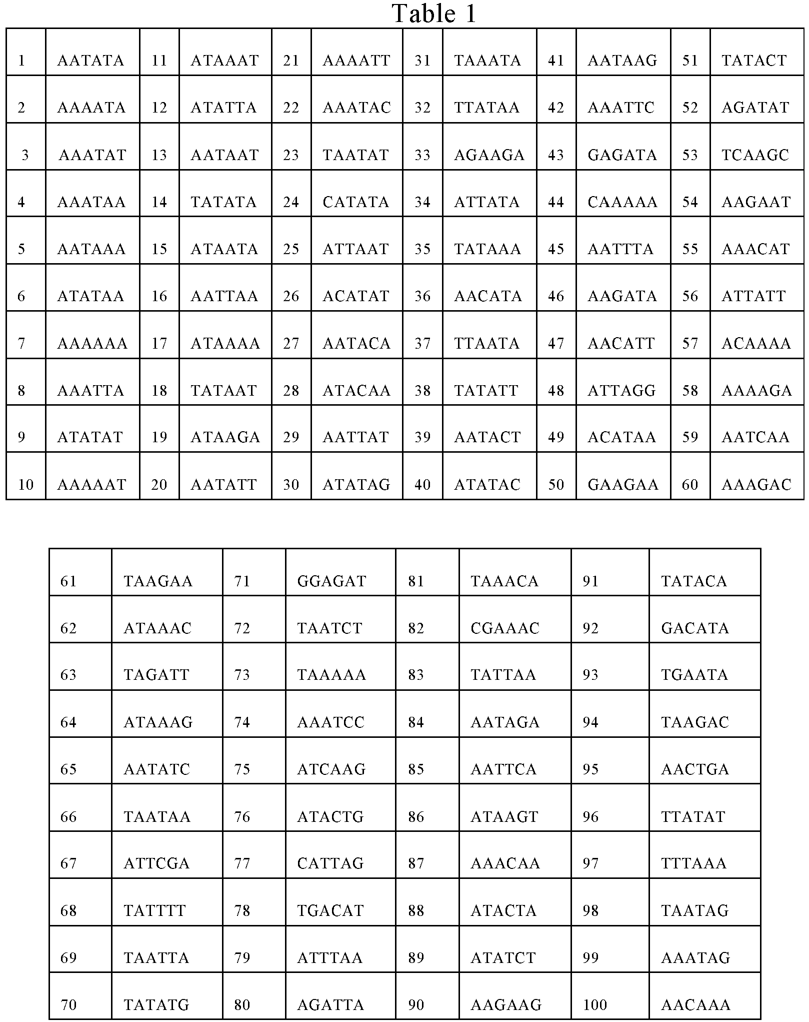

In a preferred embodiment, the coding sequence of the TI element of the present invention is shown in SEQ ID NO.: 1-40.

In the present invention, the circular RNA construct of the present invention has high translation activity and can significantly enhance translation efficiency in vivo or in vitro.

### Reaction System

The present invention provides a reaction system, including:
(a) a construct according to the first aspect of the present invention;
(b) other components required for the reaction, and the other components are selected from the group consisting of splicesomes, ribosomes, translation initiation factor EIF4G2, translation initiation factor EIF4A, translation initiation factor EIF4B, and a combination thereof.

In another preferred embodiment, the reaction system further includes YTHDF3, PABPC1, and/or hnRNPA1 protein.

In the present invention, the reaction system may be in vitro or in vivo.

### Kit

The present invention provides a kit for in vitro protein synthesis, including:
(k1) a first container, and the nucleic acid construct of claim 1 located in the first container;
(k2) a second container, and other components required for the reaction located in the second container, the other components are selected from the group consisting of spliceosome, ribosome, translation initiation factor EIF4G2, translation initiation factor EIF4A, the translation initiation factor EIF4B, and a combination thereof; and
(kt) label or instructions.

In a preferred embodiment, the first container and the second container are the same container or different containers.

### Coding sequence of foreign protein (foreign DNA)

As used herein, the terms "coding sequence of a foreign protein" and "foreign DNA" can be used interchangeably, and both refer to an exogenous DNA molecule used to direct protein synthesis. Generally, the DNA molecule is linear or circular. The DNA molecule contains a sequence encoding a foreign protein.

In the present invention, examples of the sequence encoding the foreign protein include (but are not limited to): genomic sequence, cDNA sequence. The sequence encoding the foreign protein also contains a promoter sequence, a 5' untranslated sequence, and a 3' untranslated sequence.

In the present invention, the selection of the exogenous DNA is not particularly limited. Generally, the exogenous DNA is selected from the group consisting of: an exogenous DNA encoding luciferin protein or luciferase (such as firefly luciferase), green fluorescent protein, yellow fluorescent protein, aminoacyl tRNA synthetase, glyceraldehyde-3-phosphate dehydrogenase, catalase, actin, variable regions of antibodies, DNA of luciferase mutants, and a combination thereof.

Exogenous DNA can also be selected from the group consisting of: exogenous DNA encoding α-amylase, enterocin A, hepatitis C virus E2 glycoprotein, insulin precursor, interferon alpha A, interleukin-1 beta, lysozyme, serum albumin, single-chain antibody fragment (scFV), transthyretin, tyrosinase, xylanase, and a combination thereof.

In a preferred embodiment, the foreign DNA encodes a protein selected from the group consisting of green fluorescent protein (enhanced GFP, eGFP), yellow fluorescent protein (YFP), Escherichia coli β-galactosidase (β-galactosidase, LacZ), human lysine-tRNA synthetase (Lysine-tRNA synthetase), human leucine-tRNA synthetase (Leucine-tRNA synthetase), Arabidopsis glyceraldehyde 3-phosphate dehydrogenase (Glyceraldehyde-3-phosphate dehydrogenase), mouse catalase (Catalase), and a combination thereof.

### In vitro protein synthesis method

The present invention provides an in vitro protein synthesis method, including the steps:
(i) providing the reaction system according to the second aspect of the present invention;
(ii) under a suitable condition, incubating the synthesis system of step (i) for a period of time T1, thereby synthesizing the protein.

In another preferred embodiment, the method further includes: (iii) optionally separating or detecting the protein from the reaction system.

### The main advantages of the present invention include:

(1) The present invention has first developed a set of methods for designing and synthesizing new artificially synthesized eukaryotic translation initiation elements of non-viral origin with high translation activity and controllable sequence structure and length, and this translation initiation element drives the translation of circular RNA.
(2) The present invention has screened a specific translation initiation element for the first time. The translation initiation element is very short, only 6-30 nt, but has high translation activity, and inserting the translation initiation element into the circular RNA expression vector can significantly enhance the translation efficiency both in vivo and in vitro.

The present invention will be further explained below in conjunction with specific embodiments. It should be understood that these embodiments are only used to illustrate the present invention and not to limit the scope of the present invention. The experimental methods without specific conditions in the following examples are usually based on conventional conditions, such as the conditions described in Sambrook et al., Molecular Cloning: Laboratory Manual (New York: Cold Spring Harbor Laboratory Press, 1989), or according to manufacturing The conditions suggested by the manufacturer. Unless otherwise specified, percentages and parts are weight percentages and parts by weight.

Unless otherwise specified, the materials and reagents used in the embodiments of the present invention are all commercially available products.

Taking GFP as an example of foreign protein.

### Experimental method

1. Based on the unique circular RNA reporter gene system of the present invention (the circular RNA reporter gene can be expressed under the drive of the translation initiation element to produce green fluorescent protein), a set of libraries containing millions of different sequences were constructed, screening different cell populations by cell transfection and flow cytometry (negative: no green fluorescence, positive: green fluorescence with different intensities). Performing amplicon sequencing on collected different cell populations and analyzing the sequence information contained in negative and different positive cells in combination with computational biology analysis, and extracting sequence features of different lengths from these sequence information;
2. Based on these negative and positive sequence features, through anti-neural network learning to generate translation initiation element modules of non-viral origin;
3. Inserting the generated translation initiation element modules and modular combination elements into circular RNA expression vectors, and detecting the activity of translation initiation elements by cell transfection and western blot.

### Experimental results

1. The high-throughput screening system based on circular RNA separates cell populations with different green fluorescence intensities (positive) and cell populations without fluorescence (negative).

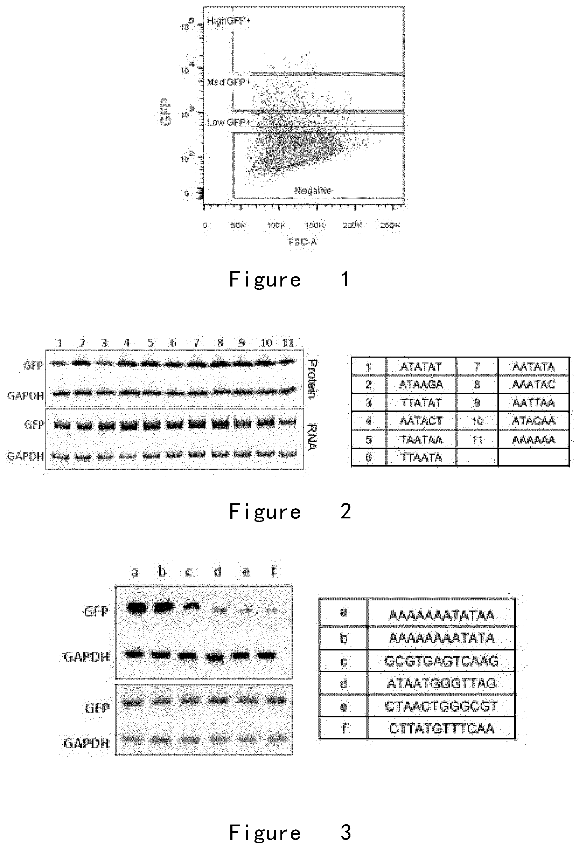

The results are shown in Table 1 and Figure 1. The results show that the circular RNA system can express green fluorescent protein and be used for screening. At the same time, the system can isolate cell populations with different fluorescence intensities, indicating that different inserted sequences in the library have a differential effect on the translation initiation of circular RNA.

2. Computational biology analysis of amplicon sequencing data of different cell populations obtained by screening and mining the sequence features contained in the high green fluorescent cell populations obtained, these sequences usually contain a large number of AU bases and fewer GC bases that can easily form RNA secondary structure. It shows that the core module of this type of translation initiation element does not depend on structure and also shows that it is significantly different from the viral IRES. It is a new class of elements that can promote non-cap-dependent translation of circular RNA, as shown in Table 1.

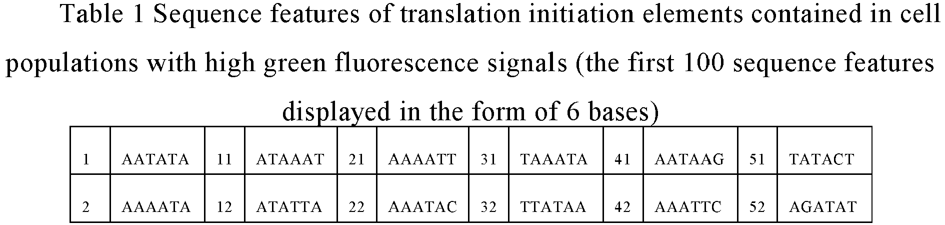

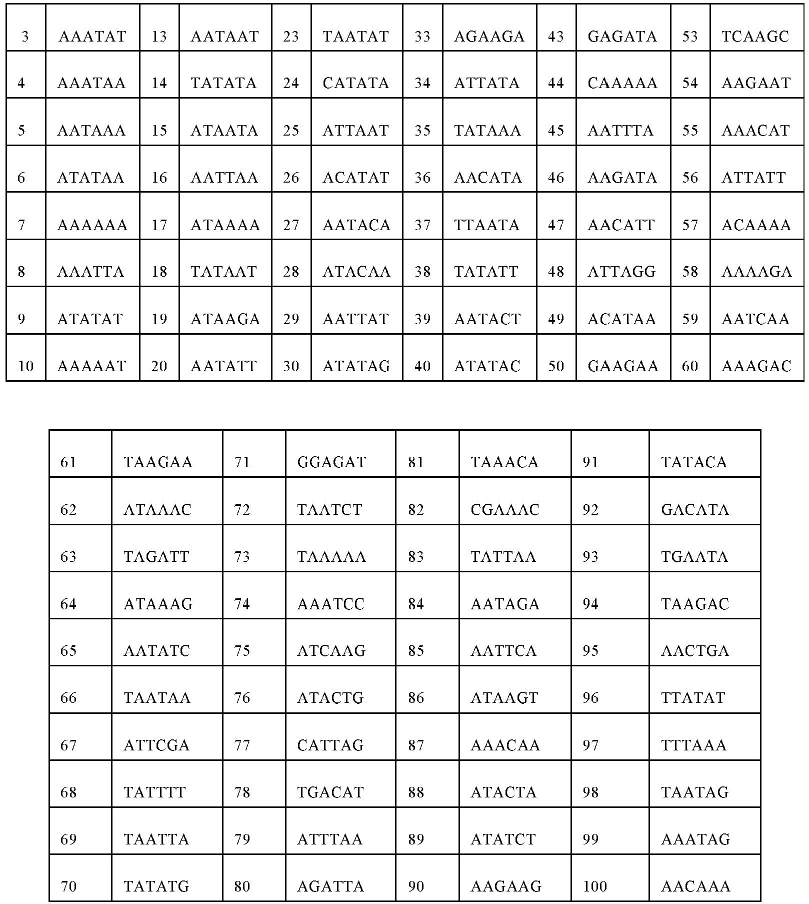

3. Detecting the translation activity of the basic feature sequence of the translation initiation element (sequence feature of 6 bases) by cell transfection and western blot, and the result is shown in Figure 2. The result shows that these translation elements all have translation initiation functions, but different sequences have different translation initiation capabilities. This indicates that based on these different sequence feature combinations, it is possible to obtain translation initiation elements with different activities.

4. The translation initiation element (taking 12 bases as an example) produced by the anti-learning based on the characteristic sequences of the positive and negative cell populations. Listing the top 20 sequences with different translational activity intensities respectively. The highly active translation initiation elements are basically AT-rich sequences, as shown in Table 2.

**Table 2 Translation initiation elements produced by anti-learning (taking 12 bases as an example)**

| SEQ ID NO.: | strongly active element | SE Q ID N O. | medium active element | SE Q ID N O. | weakly active element |
|---|---|---|---|---|---|
| 1 | AAAAAAATATAA | 21 | GCGTGAGTCAAG | 41 | CTAACTGGGCGT |
| 2 | AAAAAAAATATA | 22 | ATAATGGGTTAG | 42 | CTTATGTTTCAA |
| 3 | AAAAAAATATAT | 23 | TGACCCAACTTC | 43 | TTCCATGGACAT |
| 4 | AAAAAAAATAAA | 24 | GTAGTATTTACG | 44 | ATCAGACCGAAT |
| 5 | AAAATAAATATA | 25 | AAACATCAGTAT | 45 | AAGGAGTGACCA |
| 6 | AAAAAAAAATAT | 26 | ATTGGTGATTTG | 46 | GTCGAGCCGCTG |
| 7 | AAAAAAAAATAA | 27 | AGAATCCTAACT | 47 | ACATTGTGTGCA |
| 8 | AAAAAATATAAT | 28 | TTTAAGTACGTT | 48 | ATCACTAGGCGC |
| 9 | AAAAATATATAA | 29 | CCATACCCGGTA | 49 | GTCCCTCCGCAT |
| 10 | AAAAAATATATA | 30 | GATATTGTAGGC | 50 | GGGCCTAAGATT |
| 11 | AAAAAAATAAAT | 31 | TAGATGGATACT | 51 | GTACGCTGTTGC |
| 12 | AAAAATATATAT | 32 | TTTAACTGGGTC | 52 | AGTCTAACCCGA |
| 13 | AAAAAAAAAATA | 33 | CTGTGGATGAAC | 53 | AGGGACTTTACG |
| 14 | AAAAAAATATAG | 34 | ATGAGGAGTGGA | 54 | ACTTGCGCGTGA |
| 15 | AAATAAATATAA | 35 | GAACGGCAAAAG | 55 | GTGACAAGCGCC |
| 16 | AAAATAAATAAA | 36 | GAAGAAAGGCAC | 56 | TGACTGTTACTC |
| 17 | AAAAAAAATAAT | 37 | AATCTAACTTTT | 57 | TGAACTCCAATT |
| 18 | AAAAAATATAAA | 38 | TATCACGAATGG | 58 | CGTGCGTTTCTT |
| 19 | AAAATATATAAT | 39 | TTACATGGTCTA | 59 | CGCTACACAAAA |
| 20 | AAAATATATATA | 40 | TCCGCAGAGAAC | 60 | TCCGCCAAGTGG |

5. The translation initiation elements of different intensities obtained from anti-learning were inserted into circular RNA expression vectors, and their translation activity was detected by cell transfection and western blot. The result is shown in Figure 3. The result shows that the strong active elements in Table 2 can translate and produce more GFP protein. The medium and weakly active elements also have a certain translation efficiency, but the translation efficiency is lower than that of the strong active elements.

The result shows that the anti-learning method of the present invention can effectively predict the activity of the translation initiation element, and the method can be used to generate translation initiation elements of different lengths and intensities, and the translation initiation element of the present invention has high translation activity and can significantly enhance translation efficiency.

All publications mentioned herein are incorporated by reference as if each individual document was cited as a reference, as in the present application. It should also be understood that, after reading the above teachings of the present invention, those skilled in the art can make various changes or modifications, equivalents of which falls in the scope of claims as defined in the appended claims.

## Claims

1. A circular RNA construct which has a structure as shown in Formula I from the 5'-3' direction:
TI-Z1-Z2 (I)
wherein
TI is a translation initiation element;
Z1 is an expression cassette for expressing a foreign protein;
Z2 is none or an other component;
and, each "-" is a bond or a nucleotide connection sequence;
wherein, the length of the TI element is 6-30 nt, preferably, 8-24 nt, more preferably, 10-20 nt;
in the TI element, the content of A is ≥35%, preferably, ≥45%, more preferably, ≥60%;
in the TI element, the content of T is ≥20%, preferably, ≥30%, more preferably, ≥50%;
in the TI element, the content of A+T is ≥65%, preferably, ≥80%, more preferably, ≥90%;
in the TI element, the content of G is ≤35%, preferably, ≤25%, and more preferably, ≤10%.

2. The construct of claim 1, wherein the TI element contains one or more nucleotide sequences selected from the group consisting of as shown in Table 1:

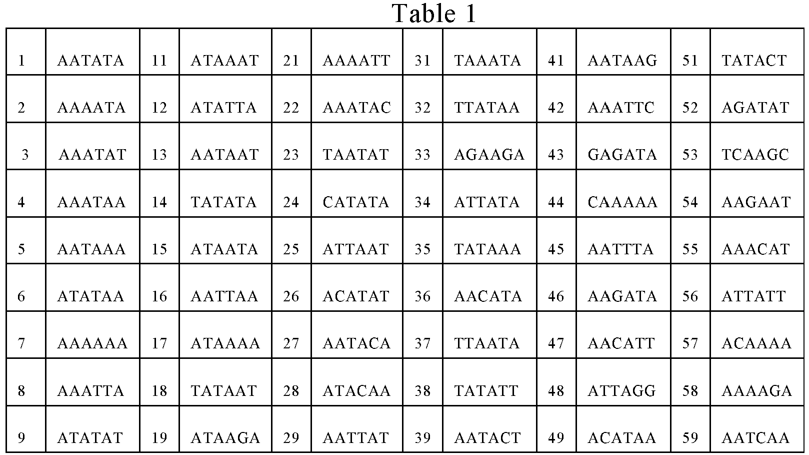

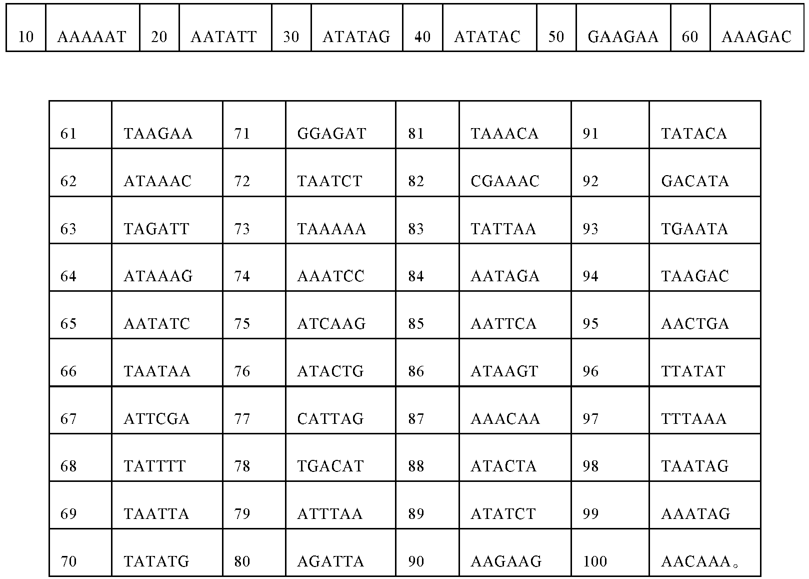

3. The construct of claim 1, wherein the coding sequence of the TI element is selected from the group consisting of;
(a) a polynucleotide whose sequence is shown in SEQ ID NO.: 1-40;
(b) a polynucleotide whose nucleotide sequence is more than 75% (preferably ≥85%, more preferably ≥90% or ≥95% or ≥98% or ≥99%) homologous to the sequence as shown in SEQ ID NO.: 1-40;
(c) a polynucleotide has 1-18 (preferably 1-10, more preferably 1-6) nucleotides truncated or added at the 5' end and/or 3' end of the polynucleotide as shown in SEQ ID NO.: 1-40;
(d) a polynucleotide complementary to any of the polynucleotides as described in (a) to (c).

4. The construct of claim 1, wherein one or more adenines (A) of the TI element are methylated.

5. A vector containing an expression cassette of the construct of claim 1.

6. A genetically engineered cell in which the nucleic acid construct of claim 1 is integrated at one or more sites of the genome of the genetically engineered cell, or the genetically engineered cell contains the vector of claim 5.

7. A reaction system, comprising:
(a) a construct of claim 1;
(b) other components required for the reaction, and the other components are selected from the group consisting of splicesomes, ribosomes, translation initiation factor EIF4G2, translation initiation factor EIF4A, translation initiation factor EIF4B, and a combination thereof.

8. A method for synthesizing protein in vitro, comprising the steps:
(i) providing the reaction system of claim 7;
(ii) under a suitable condition, incubating the synthesis system of step (i) for a period of time T1, thereby synthesizing the protein.

9. A kit for in vitro protein synthesis, comprising:
(k1) a first container, and the structure a of claim 1 located in the first container;
(k2) a second container, and other components required for the reaction located in the second container, the other components are selected from the group consisting of spliceosome, ribosome, translation initiation factor EIF4G2, translation initiation factor EIF4A, the translation initiation factor EIF4B, and a combination thereof; and
(kt) label or instructions.

10. Use of a construct of claim 1, a vector of claim 5, a genetically engineered cell of claim 6, and a reaction system of claim 7 or the kit of claim 9 for high-throughput in vitro protein synthesis.


sequence listing

<110>  SHANGHAI INSTITUTE OF NUTRITION AND HEALTH, CHINESE ACADEMY OF
SCIENCES [CN]/[CN]

<120>  PROTEIN TRANSLATION USING CIRCULAR RNA AND APPLICATION THEREOF

<130>  P2020-0291

<150>  CN 201910214234.9
<151>  2019-03-20

<160>  64

<170>  PatentIn version 3.5

<210>  1
<211>  12
<212>  DNA
<213>  artificial sequence

<220>
<223> translation initiation element

<400>  1
aaaaaaatat aa                                                          12


<210>  2
<211>  12
<212>  DNA
<213>  artificial sequence

<220>
<223> translation initiation element

<400>  2
aaaaaaaata ta                                                          12


<210>  3
<211>  12
<212>  DNA
<213>  artificial sequence

<220>
<223> translation initiation element

<400>  3
aaaaaaatat at                                                          12


<210>  4
<211>  12
<212>  DNA
<213>  artificial sequence

<220>
<223> translation initiation element

<400>  4
aaaaaaaata aa                                                          12

<210>  5
<211>  12
<212>  DNA
<213>  artificial sequence

<220>
<223> translation initiation element

<400>  5
aaaataaata ta                                                          12


<210>  6
<211>  12
<212>  DNA
<213>  artificial sequence

<220>
<223> translation initiation element

<400>  6
aaaaaaaaat at                                                          12


<210>  7
<211>  12
<212>  DNA
<213>  artificial sequence

<220>
<223> translation initiation element

<400>  7
aaaaaaaaat aa                                                          12


<210>  8
<211>  12
<212>  DNA
<213>  artificial sequence

<220>
<223> translation initiation element

<400>  8
aaaaaatata at                                                          12


<210>  9
<211>  12
<212>  DNA
<213>  artificial sequence

<220>
<223> translation initiation element

<400>  9
aaaaatatat aa                                                          12


<210>  10
<211>  12

<212>  DNA
<213>  artificial sequence

<220>
<223> translation initiation element

<400>  10
aaaaaatata ta                                                          12


<210>  11
<211>  12
<212>  DNA
<213>  artificial sequence

<220>
<223> translation initiation element

<400>  11
aaaaaaataa at                                                          12


<210>  12
<211>  12
<212>  DNA
<213>  artificial sequence

<220>
<223> translation initiation element

<400>  12
aaaaatatat at                                                          12


<210>  13
<211>  12
<212>  DNA
<213>  artificial sequence

<220>
<223> translation initiation element

<400>  13
aaaaaaaaaa ta                                                          12


<210>  14
<211>  12
<212>  DNA
<213>  artificial sequence

<220>
<223> translation initiation element

<400>  14
aaaaaaatat ag                                                          12


<210>  15
<211>  12
<212>  DNA
<213>  artificial sequence

<220>
<223> translation initiation element

<400> 15
aaataaatat aa                                                          12


<210> 16
<211> 12
<212> DNA
<213> artificial sequence

<220>
<223> translation initiation element

<400> 16
aaaataaata aa                                                          12


<210> 17
<211> 12
<212> DNA
<213> artificial sequence

<220>
<223> translation initiation element

<400> 17
aaaaaaaata at                                                          12


<210> 18
<211> 12
<212> DNA
<213> artificial sequence

<220>
<223> translation initiation element

<400> 18
aaaaaatata aa                                                          12


<210> 19
<211> 12
<212> DNA
<213> artificial sequence

<220>
<223> translation initiation element

<400> 19
aaaatatata at                                                          12


<210> 20
<211> 12
<212> DNA
<213> artificial sequence

<220>
<223> translation initiation element

<400>  20
aaaatatata ta                                                          12


<210>  21
<211>  12
<212>  DNA
<213>  artificial sequence

<220>
<223> translation initiation element

<400>  21
gcgtgagtca ag                                                          12


<210>  22
<211>  12
<212>  DNA
<213>  artificial sequence

<220>
<223> translation initiation element

<400>  22
ataatgggtt ag                                                          12


<210>  23
<211>  12
<212>  DNA
<213>  artificial sequence

<220>
<223> translation initiation element

<400>  23
tgacccaact tc                                                          12


<210>  24
<211>  12
<212>  DNA
<213>  artificial sequence

<220>
<223> translation initiation element

<400>  24
gtagtattta cg                                                          12


<210>  25
<211>  12
<212>  DNA
<213>  artificial sequence

<220>
<223> translation initiation element

<400>  25
aaacatcagt at                                                          12

<210>  26
<211>  12
<212>  DNA
<213>  artificial sequence

<220>
<223> translation initiation element

<400>  26
attggtgatt tg                                                          12


<210>  27
<211>  12
<212>  DNA
<213>  artificial sequence

<220>
<223> translation initiation element

<400>  27
agaatcctaa ct                                                          12


<210>  28
<211>  12
<212>  DNA
<213>  artificial sequence

<220>
<223> translation initiation element

<400>  28
tttaagtacg tt                                                          12


<210>  29
<211>  12
<212>  DNA
<213>  artificial sequence

<220>
<223> translation initiation element

<400>  29
ccatacccgg ta                                                          12


<210>  30
<211>  12
<212>  DNA
<213>  artificial sequence

<220>
<223> translation initiation element

<400>  30
gatattgtag gc                                                          12


<210>  31
<211>  12

<212>  DNA
<213>  artificial sequence

<220>
<223> translation initiation element

<400>  31
tagatggata ct                                                          12


<210>  32
<211>  12
<212>  DNA
<213>  artificial sequence

<220>
<223> translation initiation element

<400>  32
tttaactggg tc                                                          12


<210>  33
<211>  12
<212>  DNA
<213>  artificial sequence

<220>
<223> translation initiation element

<400>  33
ctgtggatga ac                                                          12


<210>  34
<211>  12
<212>  DNA
<213>  artificial sequence

<220>
<223> translation initiation element

<400>  34
atgaggagtg ga                                                          12


<210>  35
<211>  12
<212>  DNA
<213>  artificial sequence

<220>
<223> translation initiation element

<400>  35
gaacggcaaa ag                                                          12


<210>  36
<211>  12
<212>  DNA
<213>  artificial sequence

<220>
<223> translation initiation element

<400> 36
gaagaaaggc ac                                                          12


<210> 37
<211> 12
<212> DNA
<213> artificial sequence

<220>
<223> translation initiation element

<400> 37
aatctaactt tt                                                          12


<210> 38
<211> 12
<212> DNA
<213> artificial sequence

<220>
<223> translation initiation element

<400> 38
tatcacgaat gg                                                          12


<210> 39
<211> 12
<212> DNA
<213> artificial sequence

<220>
<223> translation initiation element

<400> 39
ttacatggtc ta                                                          12


<210> 40
<211> 12
<212> DNA
<213> artificial sequence

<220>
<223> translation initiation element

<400> 40
tccgcagaga ac                                                          12


<210> 41
<211> 12
<212> DNA
<213> artificial sequence

<220>
<223> translation initiation element

<400>  41
ctaactgggc gt                                                          12


<210>  42
<211>  12
<212>  DNA
<213>  artificial sequence

<220>
<223> translation initiation element

<400>  42
cttatgtttc aa                                                          12


<210>  43
<211>  12
<212>  DNA
<213>  artificial sequence

<220>
<223> translation initiation element

<400>  43
ttccatggac at                                                          12


<210>  44
<211>  12
<212>  DNA
<213>  artificial sequence

<220>
<223> translation initiation element

<400>  44
atcagaccga at                                                          12


<210>  45
<211>  12
<212>  DNA
<213>  artificial sequence

<220>
<223> translation initiation element

<400>  45
aaggagtgac ca                                                          12


<210>  46
<211>  12
<212>  DNA
<213>  artificial sequence

<220>
<223> translation initiation element

<400>  46
gtcgagccgc tg                                                          12

<210>  47
<211>  12
<212>  DNA
<213>  artificial sequence

<220>
<223> translation initiation element

<400>  47
acattgtgtg ca                                                          12


<210>  48
<211>  12
<212>  DNA
<213>  artificial sequence

<220>
<223> translation initiation element

<400>  48
atcactaggc gc                                                          12


<210>  49
<211>  12
<212>  DNA
<213>  artificial sequence

<220>
<223> translation initiation element

<400>  49
gtccctccgc at                                                          12


<210>  50
<211>  12
<212>  DNA
<213>  artificial sequence

<220>
<223> translation initiation element

<400>  50
gggcctaaga tt                                                          12


<210>  51
<211>  12
<212>  DNA
<213>  artificial sequence

<220>
<223> translation initiation element

<400>  51
gtacgctgtt gc                                                          12


<210>  52
<211>  12

<212>  DNA
<213>  artificial sequence

<220>
<223> translation initiation element

<400>  52
agtctaaccc ga                                                          12


<210>  53
<211>  12
<212>  DNA
<213>  artificial sequence

<220>
<223> translation initiation element

<400>  53
agggacttta cg                                                          12


<210>  54
<211>  12
<212>  DNA
<213>  artificial sequence

<220>
<223> translation initiation element

<400>  54
acttgcgcgt ga                                                          12


<210>  55
<211>  12
<212>  DNA
<213>  artificial sequence

<220>
<223> translation initiation element

<400>  55
gtgacaagcg cc                                                          12


<210>  56
<211>  12
<212>  DNA
<213>  artificial sequence

<220>
<223> translation initiation element

<400>  56
tgactgttac tc                                                          12


<210>  57
<211>  12
<212>  DNA
<213>  artificial sequence

<220>
<223> translation initiation element

<400> 57
tgaactccaa tt                                                          12


<210> 58
<211> 12
<212> DNA
<213> artificial sequence

<220>
<223> translation initiation element

<400> 58
cgtgcgtttc tt                                                          12


<210> 59
<211> 12
<212> DNA
<213> artificial sequence

<220>
<223> translation initiation element

<400> 59
cgctacacaa aa                                                          12


<210> 60
<211> 12
<212> DNA
<213> artificial sequence

<220>
<223> translation initiation element

<400> 60
tccgccaagt gg                                                          12


<210> 61
<211> 733
<212> DNA
<213> artificial sequence

<220>
<223>  circular RNA construct

<400> 61
aaaaaaaaaa atcatggtga gcaagggcga ggagctgttc accggggtgg tgcccatcct      60

ggtcgagctg gacggcgacg taaacggcca caagttcagc gtgtccggcg agggcgaggg     120

cgatgccacc tacggcaagc tgaccctgaa gttcatctgc accaccggca agctgcccgt     180

gccctggccc accctcgtga ccaccctgac ctacggcgtg cagtgcttca gccgctaccc     240

cgaccacatg aagcagcacg acttcttcaa gtccgccatg cccgaaggct acgtccagga     300

gcgcaccatc ttcttcaagg acgacggcaa ctacaagacc cgcgccgagg tgaagttcga     360

gggcgacacc ctggtgaacc gcatcgagct gaagggcatc gacttcaagg aggacggcaa 420

catcctgggg cacaagctgg agtacaacta caacagccac aacgtctata tcatggccga 480

caagcagaag aacggcatca aggtgaactt caagatccgc cacaacatcg aggacggcag 540

cgtgcagctc gccgaccact accagcagaa cacccccatc ggcgacggcc ccgtgctgct 600

gcccgacaac cactacctga gcacccagtc cgccctgagc aaagacccca acgagaagcg 660

cgatcacatg gtcctgctgg agttcgtgac cgccgccggg atcactctcg gcatggacga 720

gctgtacaag taa 733

<210> 62
<211> 1447
<212> DNA
<213> artificial sequence

<220>
<223> the circular RNA precursor

<400> 62
gactgaacat ggaggaattg aggttgggta tttcccctga ggtaggaaaa aggctgggtc 60

agtttcccgt tagccgtcaa gtcctcatca catctttaag ccttccatgc aggataaagg 120

gctgcagagc tattttcaaa ttgacatcaa actggatttc tgttgacttc gtcttccctt 180

tttaaggtcc acagaagaag atgggaagga aagaagtctg agggcatctt atttgcactc 240

cgctgtcatt tctaaggaag gcctttaatg ccaaattctc atcttttatg tccccactaa 300

atcctaaggt tcttgaactt ctgatcagac agccaaaaaa tgaaccatca actagcttaa 360

cctaacatat gtgaggatag aggactggga cagctctctg ggccactgga gagtcagaca 420

ggcctgccct ctgtgtgact tgaccgcggt ctctttcttc caggagcgca ccatcttctt 480

caaggacgac ggcaactaca agacccgcgc cgaggtgaag ttcgagggcg acaccctggt 540

gaaccgcatc gagctgaagg gcatcgactt caaggaggac ggcaacatcc tggggcacaa 600

gctggagtac aactacaaca gccacaacgt ctatatcatg gccgacaagc agaagaacgg 660

catcaaggtg aacttcaaga tccgccacaa catcgaggac ggcagcgtgc agctcgccga 720

ccactaccag cagaacaccc ccatcggcga cggccccgtg ctgctgcccg acaaccacta 780

cctgagcacc cagtccgccc tgagcaaaga ccccaacgag aagcgcgatc acatggtcct 840

gctggagttc gtgaccgccg ccgggatcac tctcggcatg gacgagctgt acaagtaaaa 900

aaaaaaaaat catggtgagc aagggcgagg agctgttcac cggggtggtg cccatcctgg 960

tcgagctgga cggcgacgta aacggccaca agttcagcgt gtccggcgag ggcgagggcg 1020

atgccaccta cggcaagctg accctgaagt tcatctgcac caccggcaag ctgcccgtgc 1080

cctggcccac cctcgtgacc accctgacct acggcgtgca gtgcttcagc cgctaccccg 1140

accacatgaa gcagcacgac ttcttcaagt ccgccatgcc cgaaggctac gtccaggtaa 1200

gtctcgacgg atcccaaata agatgccctc agacttcttt ccttcccatc ttcttctgtg 1260

gaccttaaaa agggaagacg aagtcaacag aaatccagtt tgatgtcaat ttgaaaatag 1320

ctctgcagcc ctttatcctg catggaaggc ttaaagatgt gatgaggact tgacggctaa 1380

cgggaaactg acccagcctt tttcctacct caggggaaat acccaacctc aattcctcca 1440

tgttcag 1447

<210> 63
<211> 463
<212> DNA
<213> artificial sequence

<220>
<223> first intron

<400> 63
gactgaacat ggaggaattg aggttgggta tttcccctga ggtaggaaaa aggctgggtc 60

agtttcccgt tagccgtcaa gtcctcatca catctttaag ccttccatgc aggataaagg 120

gctgcagagc tattttcaaa ttgacatcaa actggatttc tgttgacttc gtcttccctt 180

tttaaggtcc acagaagaag atgggaagga aagaagtctg agggcatctt atttgcactc 240

cgctgtcatt tctaaggaag gcctttaatg ccaaattctc atcttttatg tccccactaa 300

atcctaaggt tcttgaactt ctgatcagac agccaaaaaa tgaaccatca actagcttaa 360

cctaacatat gtgaggatag aggactggga cagctctctg ggccactgga gagtcagaca 420

ggcctgccct ctgtgtgact tgaccgcggt ctctttcttc cag 463

<210> 64
<211> 251
<212> DNA
<213> artificial sequence

<220>
<223> second intron

<400> 64
gtaagtctcg acggatccca aataagatgc cctcagactt ctttccttcc catcttcttc 60

tgtggacctt aaaaagggaa gacgaagtca acagaaatcc agtttgatgt caatttgaaa 120

atagctctgc agccctttat cctgcatgga aggcttaaag atgtgatgag gacttgacgg 180

ctaacgggaa actgacccag cctttttcct acctcagggg aaatacccaa cctcaattcc 240

tccatgttca g 251